# EUROPÄISCHE PATENTANMELDUNG

(11) **EP 0 845 266 A2**
(43) Veröffentlichungstag der Anmeldung: **03.06.1998**
(21) Anmeldenummer: 97121132.1
(22) Anmeldetag: 02.12.1997
(51) Int. Cl.: A61K 38/05, A23L 1/305, A23L 1/30

(54) **Antioxidative Zusammensetzungen mit Süssmolkekonzentrat**

(30) Priorität: 02.12.1996 DE 19649891
(71) Anmelder: MK-Produktkontor GmbH, 58642 Iserlohn (DE)
(72) Erfinder: Elstner, Erich F., Prof. Dr., 82194 Gröbenzell (DE); Krauskopf, Jobst, 58642 Iserlohn (DE)
(74) Vertreter: Hansen, Bernd, Dr. Dipl.-Chem.

(57) **Zusammenfassung**

Zusammensetzung mit bei oraler Aufnahme antioxidativer Wirkung zur pharmazeutischen oder diätetischen Verwendung, umfassend Süßmolkekonzentrat und wenigstens Anserin und/oder Carnosin sowie Verwendung der Zusammensetzung zur Herstellung eines diätetischen Lebensmittels oder einer pharmazeutischen Zubereitung.

## Beschreibung

Die gesundheitsfördernde Wirkung von Molke ist seit dem Altertum bekannt. Schon vor mehr als zweitausend Jahren wurde von Molkekuren berichtet, in Europa waren sie vor allem in der Zeit vom 17. bis 19. Jahrhundert beliebt, wobei die Förderung des allgemein Wohlbefindens durch Entgiftung und Entschlackung im Vordergrund stand. Auch im kosmetischen Bereich fand Molke Verwendung, wobei neben Molkekuren auch Molkebäder verabreicht wurden. In jüngster Zeit wurde die Molke, vor allem zur Anwendung in der modernen Diätetik, wiederentdeckt.

Molke fällt bei der Käseherstellung aus Milch, wie z.B. Kuhmilch, Ziegenmilch, Schafsmilch, Büffelmilch und Kamelmilch, an, nachdem das in der Milch vorhandene Casein ausgefällt wurde. Nach Art der Gewinnung unterscheidet man Süßmolke, die als Milchserum nach enzymatischer Ausfällung von Casein durch Labferment entsteht, und Sauermolke, die nach Abtrennung des Caseins durch Säurefällung gewonnen wird. Die pH-Wert-Grenze zwischen Süß- und Sauermolke ist nicht ganz scharf umrissen und liegt allgemein über bzw. unter einem Bereich von 5,6 bis 5,9.

Molke enthält alle wasserlöslichen Komponenten der Milch, insofern diese nicht durch Lab oder Säure ausgefällt werden. Süßmolke enthält ca. 4,9% Laktose, 0,8% Protein, 0,5% Mineralien, 0,2% Milchfett und 0,2% Milchsäure. Wichtige Proteine der Molke sind α-Lactalbumin, β-Lactoglobulin, Lactoferrin, Blutserumalbumin, Immunglobuline und Lactoperoxidase. An Fetten liegen sowohl gesättigte als auch ein- und mehrfach ungesättigte Fettsäuren sowie Cholesterin vor.

Weitere wichtige Inhaltsstoffe der Molke sind Vitamine (z.B. Ascorbinsäure, Thiamin, Niacin, Pantothensäure, Retinol, Riboflavin, Pyridoxin und Cobalamin) und freie Aminosäuren.

Süßmolke kann weiterhin teilweise oder vollständig hydrolysiert vorliegen, d.h. die Zucker und Proteine sind dann in die entsprechenden Einfachzucker bzw. Aminosäuren gespalten.

Bei allen Mengenangaben kann der Gehalt der Molke je nach verwendeter Milch (z.B. Kuh, Ziege, Schaf) und nach hergestellter Käseart schwanken.

Aufgrund ihres Nährstoffgehalts eignet sich Molke zur Herstellung ernährungsphysiologisch wertvoller Produkte. Der hohe Laktosegehalt der Molke wirkt sich günstig auf die endogene Verwertung von Calcium, Natrium und Kalium aus der Nahrung aus, und die durch Molke erhöhte Vitamin- und Mineralstoffzufuhr ist insbesondere bei älteren mangelernährten Menschen wünschenswert.

Entzündungen entstehen durch schädliche Einwirkung auf Gewebe, wie z.B. Infektionen mit Mikroorganismen. Als Symptome treten u.a. Rötung, Schwellung, Überwärmung und Schmerz auf. Verantwortlich für diese Symptome sind Mediatorstoffe von unterschiedlichem chemischem Charakter, neben biogenen Aminen (z.B. Histamin), Peptiden (z.B. Bradykinin) und Produkten des Arachidonsäurestoffwechsels (z.B. Prostaglandine, Leukotriene) gehören dazu auch reaktive Sauerstoffspezies (Superoxidradikalanionen, Wasserstoffperoxid, Hydroxylradikale, Singulettsauerstoff).

Die Bildung dieser reaktiven Sauerstoffspezies geht von polymorphkernigen Leukozyten aus, die durch Kontakt mit einem phagozytierten Mikroorganismus in einen aktivierten Zustand übergehen. In diesem stimulierten Zustand überträgt eine NADPH-Oxidase in der Plasmamembran des Leukozyten Elektronen auf Sauerstoff, wobei zunächst das Superoxidradikalanion (O₂^{·-}) gebildet wird. Durch Folgereaktionen entstehen daraus Wasserstoffperoxid (H₂O₂) und Hydroxylradikale (OH^{·}). Diese reaktiven Sauerstoffspezies werden ins Phagosom abgegeben und dienen zur Abtötung des phagozytierten Mikroorganismus. Da die Freisetzung dieser Moleküle jedoch nicht nur ins Phagosom hinein geschieht, sondern auch nach "außen", kann es zur Schädigung des umliegenden Gewebes (u.a. Lipidperoxidation, Denaturierung von Enzymen, DNA-Strangbrüche) und somit zur Entstehung von Entzündungssymptomen und Gewebedegenerationen bis hin zu Nekrosen kommen.

Neben den erwähnten reaktiven Sauerstoffspezies kann von aktivierten Leukozyten auch hypochlorige Säure (HOCl), ein starkes Oxidans, gebildet werden. Für die Bildung von HOCl ist das Enzym Myeloperoxidase (MPO) verantwortlich, welches bei der Degranulation aktivierter Leukozyten ins Phagosom und ins umliegende Gewebe abgegeben wird. Myeloperoxidase katalysiert die Umsetzung von H₂O₂ und Chlorid (Cl⁻) zu hypochloriger Säure, welche äußerst reaktiv ist und unter Chlorierung oder Oxidation mit verschiedenen Substanzgruppen reagiert (z.B. primäre Aminogruppen, Thiole, Doppelbindungen). Auch die Bildung von Singulettsauerstoff aus H₂O₂ und HOCl in einer Folgereaktion wird diskutiert.

An den oxidativen Entzündungsprozessen sind zwei Grundreaktionen beteiligt: einmal die Generierung von Superoxidradikalalanionen bzw. der Folgeprodukte Wasserstoffperoxid und Hydroxylradikale durch NADPH-Oxidasen (oder auch Xanthinoxidase), zum zweiten die Bildung von Hypochlorit und organischen Chloraminen durch Myeloperoxidase.

Die Erfinder haben festgestellt, daß die Bildung von Hypochlorit und organischen Chloraminen durch Myeloperoxidase von einer Zusammensetzung, die Süßmolkekonzentrat enthält, inhibiert wird, jedoch sind dabei relativ hohe Süßmolkekonzentrationen erforderlich, um z.B. eine 50%ige Hemmung der Hypochloritbildung zu erreichen.

Die Aufgabe der vorliegenden Erfindung liegt darin, Süßmolkekonzentrat (SMK) enthaltende Nahrungsmittel- und/oder pharmazeutische Zusammensetzungen mit bei oraler Aufnahme antioxidativer Wirkung aufzufinden, die diesen Effekt verbessern.

Es wurde überraschend gefunden, daß die Zusammensetzung gemäß Anspruch 1, die SMK und eines oder beide der Dipeptide Anserin und Carnosin umfaßt, die Hypochloritbildung in stärkerem Maße hemmt, als dies aus der Summe der Wirkungen von SMK bzw. Anserin und/oder Carnosin allein zu erwarten wäre. Da die Dosis eines Wirkstoffs das Produkt aus dessen Konzentration und der Einwirkungszeit ist, wird als ein wesentliches Merkmal SMK und nicht Süßmolke als solche in der erfindungsgemäßen Zusammensetzung verwendet. Speziell durch SMK können genügend hohe Konzentrationen der wirksamen Bestandteile gebildet werden, insbesondere bei Einwirkung und Passage durch den Mundraum, um die erwünschte Wirkung entstehen zu lassen, die mit Süßmolke in üblicher Konzentration nicht beobachtbar ist.

Durch Zusatz wenigstens einer der Substanzen Taurin, Hypotaurin oder Lactobionat kann die Hemmwirkung der Zusammensetzung noch gesteigert werden. In einer weiteren Ausführungsform umfaßt die erfindungsgemäße Zusammensetzung weitere, zur Herstellung von diätetischen Lebensmitteln geeignete Zusatzstoffe. In einer weiteren Ausführungsform umfaßt die in den Ansprüchen 1 und 2 beschriebene Zusammensetzung zusätzlich pharmazeutisch annehmbare Zusatzstoffe und/oder Trägermaterialien.

Die erfindungsgemäße Zusammensetzung kann zur Herstellung von Lebensmitteln, wie z.B. diätetischen Lebensmitteln, und Nahrungsergänzungsmitteln verwendet werden. Sie kann weiterhin zur Herstellung einer pharmazeutischen Zusammensetzung zur Behandlung von Erkrankungen, die mit oxidativen Prozessen verbunden sind, verwendet werden, bevorzugt zur Behandlung von Entzündungserkrankungen von Säugern, insbesondere des Menschen. Noch mehr bevorzugt ist die Verwendung zur Behandlung von Entzündungserkrankungen der epithelialen Kontakt- und Abwehrflächen, insbesondere des Mund-Rachenbereiches, des Nasen-Ohrenbereiches, der Atemwege und der Verdauungswege, sowie des Genitalbereich, insbesondere in der Frauenheilkunde.

Die erfindungsgemäße Zusammensetzung kann schließlich in verschiedenen Formen, z.B. in fester Form als Lutschtablette, Pulver oder Granulat, oder in flüssiger Form als Sirup oder Saft vorliegen.

Die Dosierung der erfindungsgemäßen Zusammensetzung kann je nach Alter, Gewicht und Geschlecht der zu behandelnden Person variieren. Bei der Verabreichung der erfindungsgemäßen Zusammensetzung an Säuger im allgemeinen erfolgt die Dosierung ebenfalls in Abhängigkeit von Tierart und Gewicht.

### Beispiel

Die Inhibitionswirkung wurde in einem In-vitro-Teststystem geprüft, das die oxidativen Prozesse während eine Entzündung simulieren soll.

Myeloperoxidase (MPO) wird von aktivierten Leukozyten während der Degranulation in das Phagosom und das umliegende Gewebe abgegeben. Das Enzym katalysiert die Bildung von HOCl aus H₂O₂ und Chlorid. Durch Messung der HOCl-Konzentration in dem Reaktionssystem kann das Ausmaß der Inhibition bestimmt werden. Hypochlorit (HOCl) spaltet spezifisch Aminocyclopropancarbonsäure (ACC), wobei Ethylen entsteht.

Dieses wiederum kann gaschromatographisch identifiziert werden.

Der Einfluß einer Mischung aus Süßmolkekonzentrat (SMK) und Anserin bzw. Carnosin sowie der einzelnen Komponenten in den in Tabelle 1 angegebenen Konzentrationen auf die MPO-katalysierte Bildung von HOCl wurde untersucht.

**Tabelle 1**

| Einfluß von Anserin (AN) und Carnosin (CAR) in Abwesenheit oder Anwesenheit von Süßmolkekonzentrat (SMK, 3 mg im Ansatz) auf die Myeloperoxidase-Reaktion. | | | | | |
|---|---|---|---|---|---|
| Konzentration AN, CAR (mM) | AN | CAR | AN+SMK | CAR+SMK | SMK Effekt (%) |
| | (% Hemmung) | | | | |
| 0,0 | 0 | 0 | 20 | 20 | 20 |
| 0,1 | 23 | 23 | 64 | 65 | 42 |
| 1,0 | 84 | 86 | 87 | 88 | 3 |

Es wurde folgender Ansatz verwendet: 0,15 M NaCl, 25 µM H₂O₂, 0,1 E MPO, 0,1 M Phosphatpuffer mit einem pH-Wert von 5,7 und 1 mM ACC. Die Inkubationsdauer beträgt 30 Minuten bei 37 °C. Die Ethylen-Freisetzungsrate eines Ansatzes ohne Zugabe potentieller Inhibitorstoffe entspricht einer Grundrate von 100% (0% Hemmung).

Aus der Tabelle ist ersichtlich, daß der Inhibitionseffekt der Mischung aus SMK und Anserin bzw. Carnosin bei mikromolaren Konzentrationen deutlich höher ist als die Summe der Inhibitionseffekte der einzelnen Bestandteile. Es kann also nicht von einem rein additiven Effekt der getesteten Einzelsubstanzen gesprochen werden, sondern vielmehr zeigt die Mischung einen überraschenden synergistischen Effekt.

Das oben beschriebene In-vitro-Testsystem zeigt, daß die antioxidative Wirkung von Süßmolke in einer Zusammensetzung, die neben Süßmolke auch Anserin und/oder Carnosin umfaßt, in deutlich überadditiver Weise zunimmt.

## Patentansprüche

1. Zusammensetzung mit bei oraler Aufnahme antioxidativer Wirkung zur pharmazeutischen oder diätetischen Verwendung, umfassend Süßmolkekonzentrat und wenigstens ein Dipeptid ausgewählt aus Anserin und Carnosin.

2. Zusammensetzung mit bei oraler Aufnahme antioxidativer Wirkung zur pharmazeutischen oder diätetischen Verwendung, umfassend Süßmolkekonzentrat, wenigstens ein Dipeptid ausgewählt aus Anserin und Carnosin sowie wenigstens eine Substanz ausgewählt aus Taurin, Hypotaurin und Lactobionat.

3. Zusammensetzung gemäß Anspruch 1 oder 2, wobei das Süßmolkekonzentrat ein Konzentrat aus teilweise oder vollständig hydrolysierter Süßmolke ist.

4. Zusammensetzung gemäß einem der Ansprüche 1-3, wobei das Süßmolkekonzentrat aus Kuhmilch, Ziegenmilch, Schafsmilch, Büffelmilch oder Kamelmilch, gewonnen wird.

5. Zusammensetzung gemäß einem der Ansprüche 1-4, wobei die Zusammensetzung ferner Zusatzstoffe enthält, die zur Herstellung von Lebensmitteln, insbesondere diätetischen Lebensmitteln, und Nahrungsergänzungsmitteln geeignet sind.

6. Zusammensetzung gemäß einem der Ansprüche 1-5, wobei die Zusammensetzung ferner pharmazeutisch annehmbare Zusatzstoffe und/oder Trägermaterialien umfaßt.

7. Zusammensetzung gemäß einem der Ansprüche 1-6, wobei die Zusammensetzung in fester Form als Lutschtablette, Pulver oder Granulat, oder in flüssiger Form als Sirup oder Saft vorliegt.

8. Verwendung der Zusammensetzung gemäß einem der Ansprüche 1-6 zur Herstellung eines diätetischen Lebensmittels.

9. Verwendung der Zusammensetzung gemäß einem der Ansprüche 1-6 zur Herstellung einer pharmazeutischen Zusammensetzung zur Behandlung von Erkrankungen, die mit oxidativen Prozessen verbunden sind, bei Säugern.

10. Verwendung der Zusammensetzung gemäß Anspruch 9, wobei die Zusammensetzung zur Behandlung von Erkrankungen beim Menschen verwendet wird.

11. Verwendung der Zusammensetzung gemäß Anspruch 9 oder 10, wobei die Erkrankungen Entzündungen sind.

12. Verwendung der Zusammensetzung gemäß Anspruch 9 oder 10, wobei die Erkrankungen Entzündungen der epithelialen Kontakt- und Abwehrflächen, insbesondere des Mund-Rachenbereiches, des Nasen-Ohrenbereiches, der Atemwege und der Verdauungswege, sind.

13. Verwendung der Zusammensetzung gemäß Anspruch 10, wobei es sich um entzündliche Erkrankungen im Genitalbereich, insbesondere in der Frauenheilkunde, handelt.
